# EUROPÄISCHE PATENTSCHRIFT

(11) **EP 1 310 231 B1**
(45) Veröffentlichungstag und Bekanntmachung des Hinweises auf die Patenterteilung: **11.02.2004**
(21) Anmeldenummer: 01126486.8
(22) Anmeldetag: 12.11.2001
(51) Int. Cl.: A61K 7/13

(54) **Verfahren zum Färben von menschlichen Haaren**
Process for dyeing human hair
Procédé de teinture pour cheveux humains

(43) Veröffentlichungstag der Anmeldung: 14.05.2003
(73) Patentinhaber: KPSS-Kao Professional Salon Services GmbH, 64297 Darmstadt (DE)
(72) Erfinder: Möhring, Hartmut, Dr., 64342 Seeheim-Jugenheim (DE); Pratt, Dominic, Dr., 64295 Darmstadt (DE)

(56) Entgegenhaltungen:
- EP-A- 0 624 362
- DE-A- 19 731 166
- DE-A- 19 949 033
- US-A- 3 396 736
- US-A- 3 679 347
- US-A- 4 102 641

## Beschreibung

Die Erfindung betrifft ein neuartiges Verfahren zum Färben von menschlichen Haaren, das langanhaltende Färbungen mit guten Farbeigenschaften wie Licht-, Wasch- und Umweltstabilität ergibt, wobei auch bei wiederholter Anwendung keine Haarschädigung auftritt.

Bei der Haarfärbung finden grundsätzlich zwei Verfahren Anwendung:

Einerseits die Färbung mit direktziehenden Farbstoffen, die zwar das Haar auch bei wiederholter Anwendung nicht schädigt, aber zu Färbungen führt, die nicht sehr langlebig sind und auch bei wiederholter Haarwäsche und durch Umwelteinflüsse verblassen, da die verwendeten direktziehenden Farbstoffe nur oberflächlich auf das Haar aufziehen.

Alternativ dazu lassen sich durch die Anwendung von Oxidationsfarbstoffzubereitungen, die Oxidationsfarbstoffvorprodukte und Oxidationsmittel enthalten, dauerhafte Haarfärbungen erzielen, da dabei eine Reaktion direkt mit den Haaren stattfindet. Bei kurzfristig wiederholter Anwendung können jedoch, insbesondere bei empfindlichen Haaren, Haarschädigungen auftreten.

Die Erfindung geht von der Aufgabenstellung aus, die Nachteile beider Verfahren zu vermeiden, und eine dauerhafte Haarfärbung ohne jedwede Haarschädigung zu bewirken.

Die Lösung dieser Aufgabe besteht in einem neuen Verfahren zum Färben von menschlichen Haaren, das durch die Kombination folgender Verfahrensschritte gekennzeichnet ist:
a) Aufbringen einer Zusammensetzung mit einem pH-Wert von 2 bis 5, enthaltend mindestens einen Reaktivfarbstoff, auf menschliches Haar;
b) Einwirkenlassen zwischen etwa 5 bis 45 Minuten;
c) gegebenenfalls Spülen und Trocknen;
d) Aufbringen einer alkalischen, mindestens ein Oxidationsfarbstoff-Vorprodukt und ein Peroxid enthaltenden Zusammensetzung mit einem pH-Wert von 7,5 bis 12;
e) Einwirkenlassen für 1 bis 20 Minuten; und
f) Ausspülen bzw. Auswaschen und Trocknen des gefärbten Haares.

Dadurch wird eine dauerhafte, gegenüber Umwelteinflüssen und Haarwäschen stabile Haarfärbung erzielt, ohne irgendeine Haarschädigung zu bewirken.

Aus der Parallelanmeldung (A-31/00) ist ein analoges Verfahren bekannt, umfassend
a) das Aufbringen einer Zusammensetzung mit einem pH-Wert von 2 bis 5, enthaltend mindestens einen Reaktivstoff, auf menschliches Haar
b) Einwirkenlassen zwischen etwa 5 bis 45 Minuten;
c) gegebenenfalls Spülen und Trocknen;
d) Aufbringen einer alkalischen Zusammensetzung mit einem pH-Wert von 7,5 bis 12;
e) Einwirkenlassen für 1 bis 20 Minuten; und
f) Ausspülen bzw. Auswaschen und Trocknen des gefärbten Haares

Reaktivfarbstoffe sind seit langem bekannt; sie weisen neben einer farbgebenden Komponente mindestens eine reaktive Gruppe im Molekül auf, die durch Reaktion mit funktionellen Gruppen des Substrats, im vorliegenden Fall das Haarkeratin, an dieses gebunden wird.
In der Regel enthalten diese Reaktivstoffe, die beispielsweise in Römpp Chemie Lexikon, 9. Aufl., S. 3805-3806, mit ausführlichen Literaturhinweisen definiert sind, auch noch hydrophile Gruppen.
Erfindungsgemäß werden bevorzugt Reaktivfarbstoffe eingesetzt, die entweder mindestens eine Vinylsulfongruppe aufweisen, oder mindestens eine Sulfatoethylsulfongruppe enthalten, die Sulfat abspalten kann und in die Vinylsulfongruppe übergeht, die dann mit reaktiven Gruppen des Haarkeratins reagiert.

Besonders bevorzugte Reaktivfarbstoffe sind solche der Formeln:

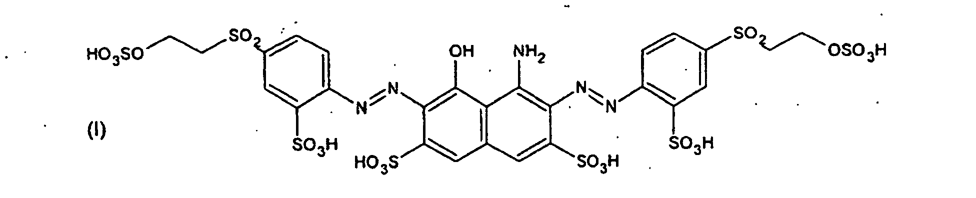

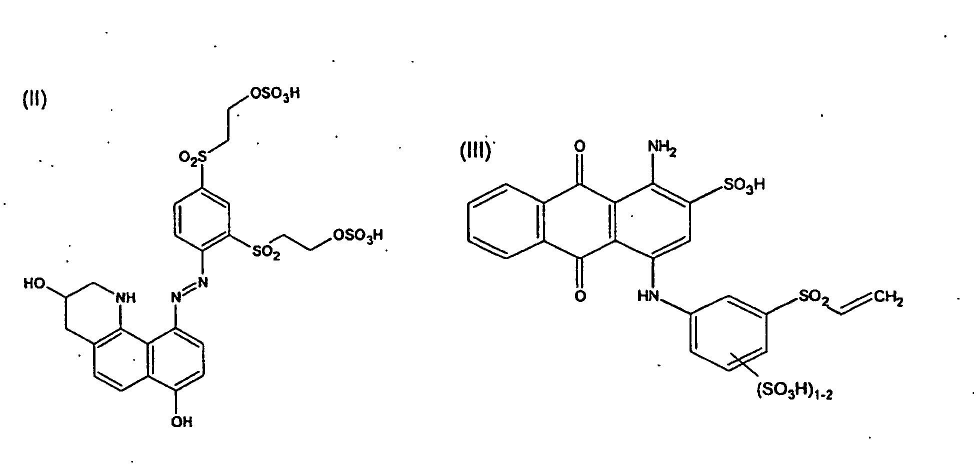

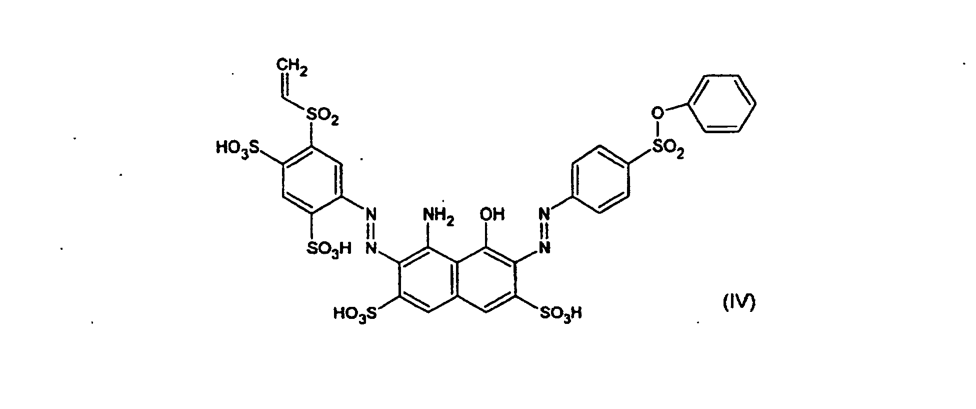

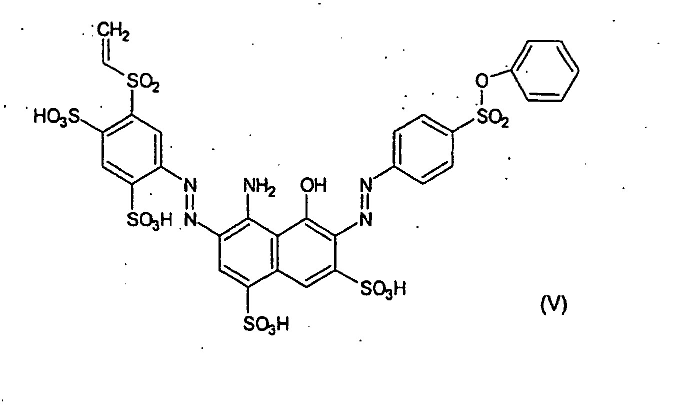

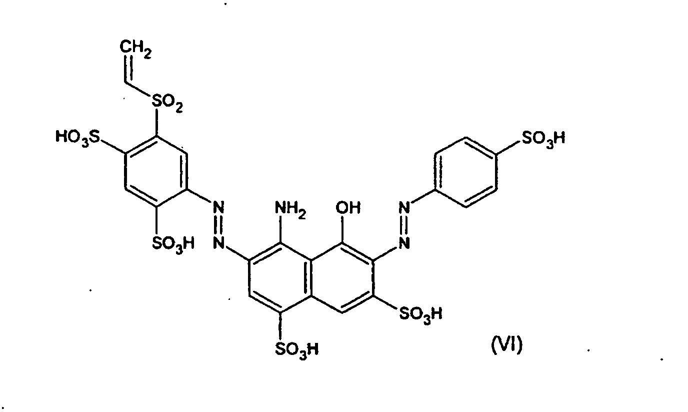

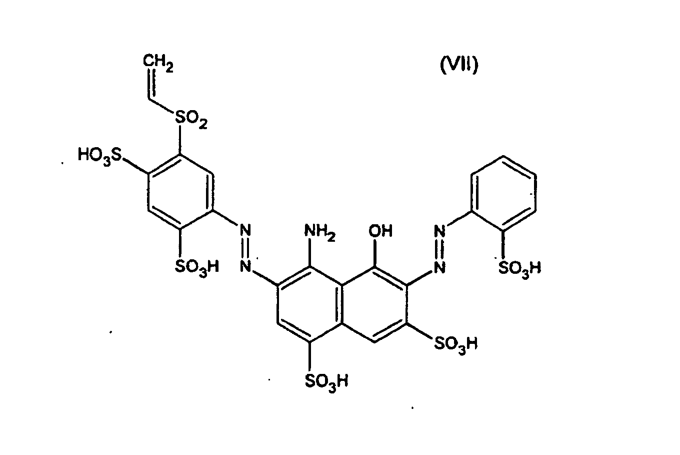

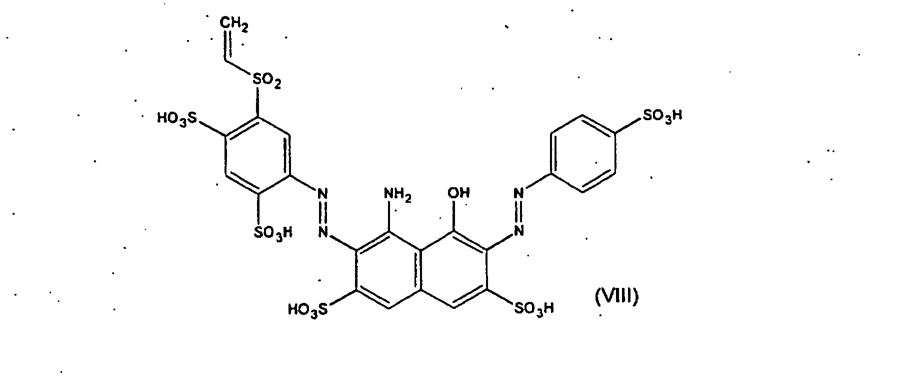

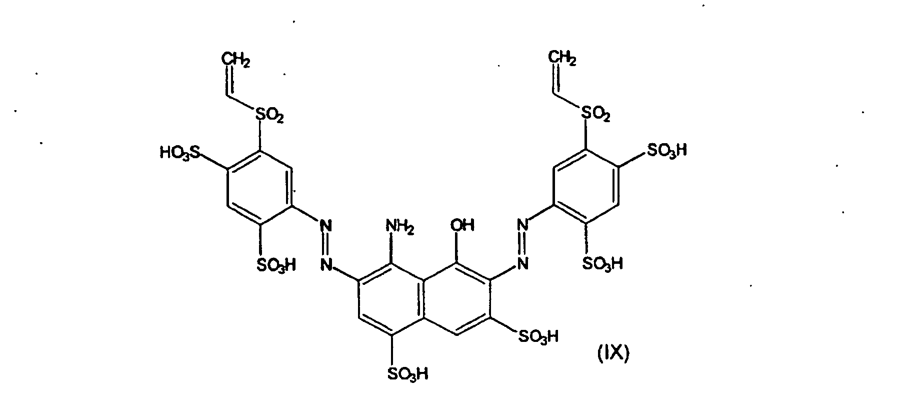

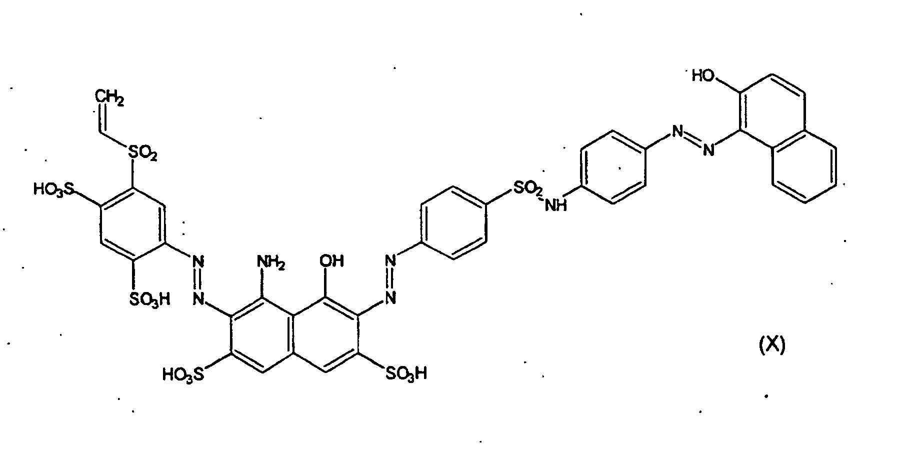

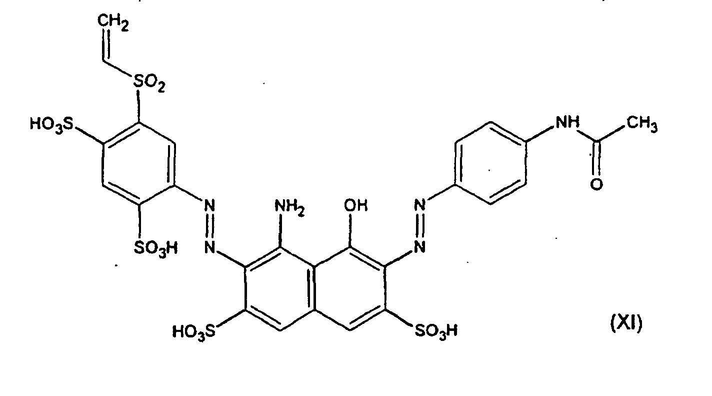

Geeignet sind daneben auch Reaktivfarbstoffe mit halogenhaltigen, heterocyclischen Resten wie Triazinen, Pyrazinen, Pyrimidinen, etc.
Als farbgebende Gruppen sind beispielsweise Diazogruppen, Anthrachinon-, Phthalocyanin- und Dioxazin-Strukturen geeignet.

Zur Applikation können die Reaktivfarbstoffe in allen zur Haarfärbung benutzten Medien angewandt werden, insbesondere Lösungen, Emulsionen, Gelen oder (Schaum-)Aerosolen, die die in solchen Zusammensetzungen üblichen Stoffe wie oberflächenaktive Mittel, Verdickungsmittel, Riechstoffe, Stabilisatoren, Lösungsvermittler, natürliche und/oder synthetische Polymere, Komplexbildner, etc. enthalten; die Zusammensetzung dieser Trägermaterialien ist grundsätzlich bekannt und beispielsweise in der Monographie von K. Schrader, Grundlagen und Rezepturen der Kosmetika, 2. Aufl. (1989), S. 796-798, beschrieben.
Der pH-Wert der Zusammensetzung liegt zwischen 2 und 5, vorzugsweise etwa 2,5 bis 4, und wird auf an sich bekannte Weise eingestellt.

Die Konzentration der Reaktivfarbstoffe in der auf das Haar applizierten Zusammensetzung ist abhängig von der Struktur derselben und der erwünschten Farbintensität; sie liegt im allgemeinen bei etwa 0,01 bis etwa 5, insbesondere etwa 0,05 bis 2,5, vorzugsweise etwa 0,1 bis etwa 1 Gew.-%.
Dabei können zur Nuancierung der Färbung auch direktziehende Farbstoffe zugesetzt werden.

Die Einwirkungszeit dieser sauren Reaktivfarbstoff-Zusammensetzung liegt vorzugsweise bei etwa 10 bis 30 Minuten; sie kann durch Wärmeeinwirkung vermindert werden.

Zwischen die Verfahrensschritte b) und d), d.h. Einwirkung der sauren Reaktivfarbstoff-Zusammensetzung und Aufbringung der alkalischen Oxidationsfarbstoffzusammensetzung, kann ein weiterer Arbeitsgang c) eingeschaltet werden, der eine zwischenzeitliche Spülung und gegebenenfalls Trocknung des Haares beinhaltet.

Die im Anschluß an den Verfahrensschritt b) und, falls fakultativ durchgeführt, c) aufzubringende alkalische Oxidationsfarbstoffzusammensetzung weist einen pH-Wert von 7,5 bis 12, vorzugsweise etwa 8 bis 11, insbesondere etwa 8,5 bis 10 auf. Bevorzugte Alkalisierungsmittel darin sind Ammoniak, Monoethanolamin sowie Alkalicarbonate und -hydrogencarbonate wie Natrium- und Kaliumcarbonat und -hydrogencarbonat.
Grundsätzlich können jedoch auch andere Alkalisierungsmittel wie Di- und Triethanolamin sowie weitere Hydroxyalkanolamine eingesetzt werden. Die Einwirkungszeit der alkalischen Zusammensetzung auf dem Haar beträgt etwa 1 bis 20, vorzugsweise etwa 5 bis 15 Minuten.
Danach wird das Haar gewaschen bzw. gespült und getrocknet.
Diese alkalische Zusammensetzung kann ebenfalls als Lösung, Emulsion, Dispersion, Gel oder (Schaum-)Aerosol vorliegen und weitere Bestandteile, z.B. pflegende oder konditionierende Substanzen, enthalten.
Diese Bestandteile sind dem Fachmann an sich bekannt und bedürfen daher keiner weiteren Erläuterungen.

Die Oxidationsfarbstoff-Zusammensetzungen enthalten üblicherweise mindestens eine Entwickler- und mindestens eine Kupplersubstanz und werden vor der Anwendung mit einer Wasserstoffperoxidlösung, beispielsweise mit einem Gehalt an 6 Gew.-% H₂O₂, im Gewichtsverhältnis 1:1 vermischt.

Bezüglich der alkalisch eingestellten gebrauchsfertigen (d.h., das Peroxid enthaltenden) Mischungen, deren pH-Wert vorzugsweise zwischen etwa 8 und 11, insbesondere 9 und 10, beispielsweise bei etwa 9,5 liegt, wird hierzu auf den Stand der Technik, z.B. auf die Monografie von K. Schrader, "Grundlagen und Rezepturen der Kosmetika", 2. Aufl., S. 784-804 (1989), verwiesen; die dort beschriebenen Produkte sind im Rahmen des erfindungsgemäßen Verfahrens ebenso einsetzbar wie die aus dem umfangreichen Stand der Technik bekannten weiteren Entwicklerund Kupplersubstanzen sowie Nuanceure.

Beispielhafte Entwicklersubstanzen sind insbesondere 1,4-Diaminobenzol, 2,5-Diaminotoluol, Tetraaminopyrimidine, Triaminohydroxypyrimidine, 1,2,4-Triaminobenzol, 2-(2,5-Diaminophenyl)ethanol, 2-(2-Hydroxyethylamino)-5-amino-toluol und 1-Amino-4-bis-(2'-hydroxyethyl)aminobenzol bzw. deren wasserlösliche Salze; beispielhafte Kupplersubstanzen sind Resorcin, 2-Methylresorcin, 4-Chlorresorcin, 2-Amino-4-chlorphenol, 4-(N-methyl)aminophenol, 2-Aminophenol, 3-Aminophenol, 1-Methyl-2-hydroxy-4-aminobenzol, 3-N,N-Dimethylaminophenol, 4-Amino-3-methylphenol, 5-Amino-2-methylphenol, 6-Amino-3-methylphenol, 3-Amino-2-methylamino-6-methoxypyridin, 2-Amino-3-hydroxypyridin, 4-Aminodiphenylamin, 4,4'-Diaminodophenylamin, 2-Dimethylamino-5-aminopyridin, 2,6-Diaminopyridin, 1,3-Diaminobenzol, 1-Amino-3-(2'-hydroxyethylamino)benzol, 1-Amino-3-[bis(2'-hydroxyethyl)amino]benzol, α-Naphthol, 1,4-Diamino-2-chlorbenzol, 4,6-Dichlorresorcin, 1,3-Diaminotoluol, 1-Hydroxynaphthalin, 4-Hydroxy-1,2-methylendioxy-benzol, 1,5-Dihydroxynaphthalin, 1,7-Dihydroxynaphthalin, 2,7-Dihydroxynaphthalin, 4-Hydroxy-1,2-methylendioxybenzol, 2,4-Diamino-3-chlorphenol und/oder 1-Methoxy-2-amino-4-(2'-hydroxyethylamino)benzol, ohne daß diese beispielhafte Aufzählung Anspruch auf Vollständigkeit erheben könnte.

Entwickler- und Kupplersubstanzen sind vorzugsweise im Molverhältnis 1:3 bis 5:1, insbesondere etwa 1:1 und etwa 3:1, enthalten; ihr Anteil in den erfindungsgemäß eingesetzten Farbzusammensetzungen kann jeweils etwa 0,25 bis etwa 5 Gew.-%, je nach gewünschter Färbung, betragen.

Als Oxidationsmittel werden vor allem verdünnte Wasserstoffperoxid-Lösungen, -Emulsionen oder -Gele eingesetzt; möglich, aber weniger üblich ist auch die Verwendung weiterer Peroxide wie Erdalkaliperoxiden, Harnstoffperoxid, Melaminperoxid, etc. in entsprechenden stöchiometrischen Mengen.

Die Oxidationsfarbstoffzusammensetzungen können als Lösungen, Cremes, Pasten, Gele, Aerosole, etc. Verwendung finden.

Die folgenden Beispiele beschreiben einige Aspekte der Erfindung.

Die Verwendung einer alkalischen Zusammensetzung ohne Oxidationsfarbstoff-Vorprodukte im Beispiel ist nicht erfindungsgemäß, sondern dient zur Erläuterung.

### Beispiele

### 1. Methode

Haarsträhnen aus unbehandeltem, weißen Ziegenhaar wurden angefeuchtet; anschließend wurde jeweils eine 0,1%-ige wäßrige Lösung (pH3) der unter 3. definierten Reaktivfarbstoffe gleichmäßig aufgetragen.

Nach jeweils 15-minütiger Einwirkung wurde, mit Wasser gespült getrocknet und anschließend eine alkalische Mischung der weiter unten definierten Zusammensetzungen aufgetragen.
Nach erfolgter 10-minütiger Einwirkung bei Raumtemperatur wurde wieder gespült und getrocknet.
Es wurden sowohl die Farbintensität visuell als auch die Licht- und Waschstabilität der erzielten Färbung am Haar im Vergleich zu nicht erfindungsgemäß behandelten Haarsträhnen, d.h. solchen, bei denen keine alkalische Nachbehandlung durchgeführt worden war, untersucht.
Die 72-stündige Extraktion durch eine 50%-ige wäßrige Pyridinlösung ist ein Maß für die Menge des Farbstoffs, der mit dem Haarkeratin reagiert hat, d.h., je mehr Farbstoff mittels Pyridin extrahiert wird, desto niedriger der Bindungsgrad und damit die Farbwirkung.

### 2. Untersuchte Zusammensetzungen:

### Saure Farbstofflösungen

### a) Trägerlösung:

| | |
|---|---|
| 25,0 (Gew.-%) | Propylencarbonat |
| 5,0 | Ethanol |
| 3,0 | PEG-40 Hydriertes Ricinusöl |
| 5,0 | Milchsäure |
| 0,3 | NaOH, 32%-ig |
| 0,1 | Reaktivfarbstoff |
| ad 100,0 | Wasser |

### b) Alkalische Oxidationsfarbstoff-Zusammensetzungen

### Trägermasse

| | |
|---|---|
| Cetylstearylalkohol | 11,00 (Gew.%) |
| Oleth-5 | 5,00 |
| Ölsäure | 2,50 |
| Stearinsäuremonoethanolamid | 2,50 |
| Cocosfettsäuremonoethanolamid | 2,50 |
| Natriumlaurylsulfat | 1,70 |
| Natriumsulfit | 1,00 |
| 1,2-Propandiol | 1,00 |
| Ascorbinsäure | 0,50 |
| Ammoniumchlorid | 0,50 |
| EDTA, Tetranatriumsalz | 0,20 |
| Parfum | 0,40 |
| Weizenproteinhydrolysat | 0,20 |
| Silica | 0,10 |
| Ammoniak (25%) | 7,20 |
| Wasser | ad 100,00 |

Vor der Anwendung wurden diese Mischungen jeweils mit einer 6%-igen H₂O₂-Zusammensetzung im Gewichtsverhältnis 1:1 gemischt.

### 3. Ergebnisse

| Reaktivfarbstoff (Allgemeine Formel) | Extrahierbar aus dem Haar | Farbe |
|---|---|---|
| III | 10% | glänzendes Blau |
| VIII | 12% | dunkles Blauschwarz |

## Patentansprüche

1. Verfahren zum Färben von menschlichen Haaren, **gekennzeichnet durch** die Kombination folgender Verfahrensschritte:
a) Aufbringen einer Zusammensetzung mit einem pH-Wert von 2 bis 5, enthaltend mindestens einen Reaktivfarbstoff, auf menschliches Haar;
b) Einwirkenlassen zwischen etwa 5 bis 45 Minuten;
c) gegebenenfalls Spülen und Trocknen;
d) Aufbringen einer alkalischen, mindestens ein Oxidationsfarbstoff-Vorprodukt und ein Peroxid enthaltender Zusammensetzung mit einem pH-Wert von 7,5 bis 12;
e) Einwirkenlassen für 1 bis 20 Minuten; und
f) Ausspülen bzw. Auswaschen und Trocknen des gefärbten Haares.

2. Verfahren nach Anspruch 1, **dadurch gekennzeichnet, daß** die in der Verfahrensstufe a) aufgebrachte Zusammensetzung mindestens einen Reaktivfarbstoff der folgenden Formeln 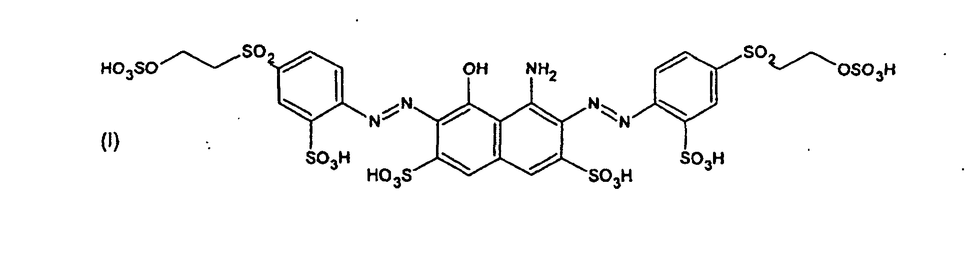 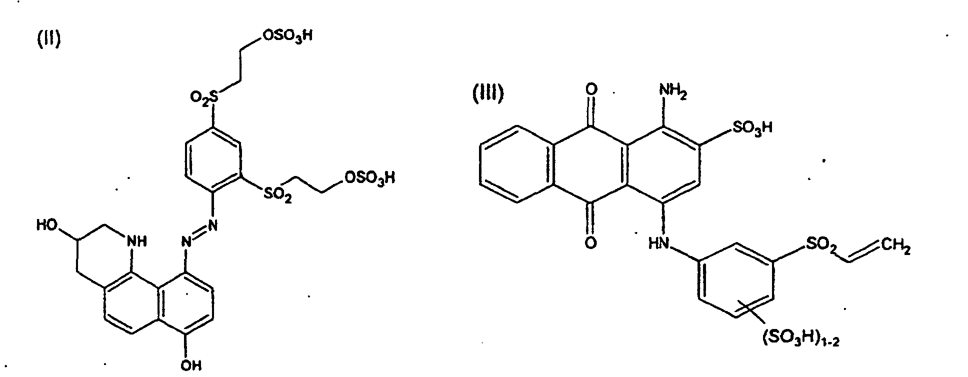 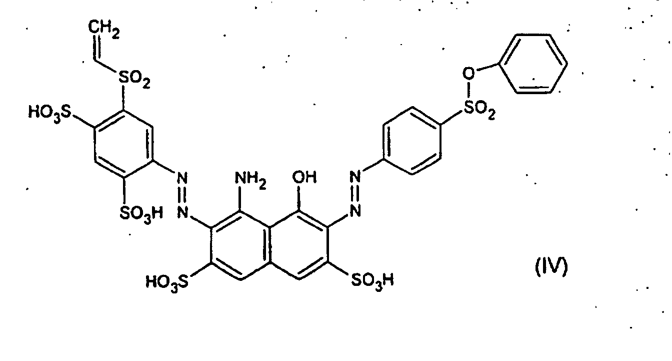 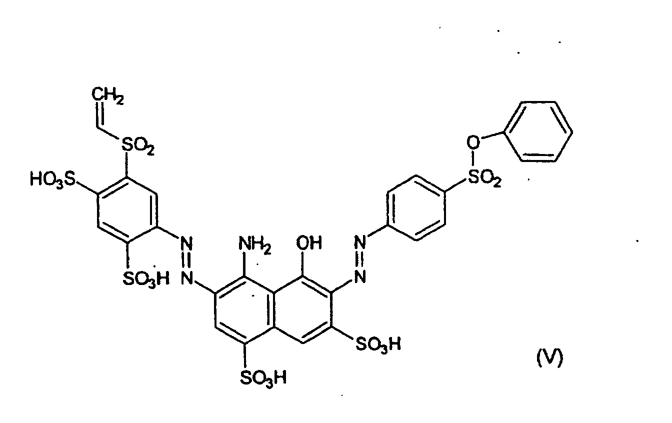 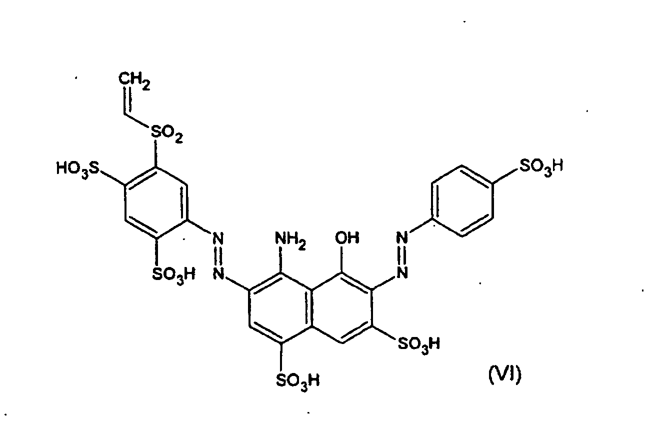 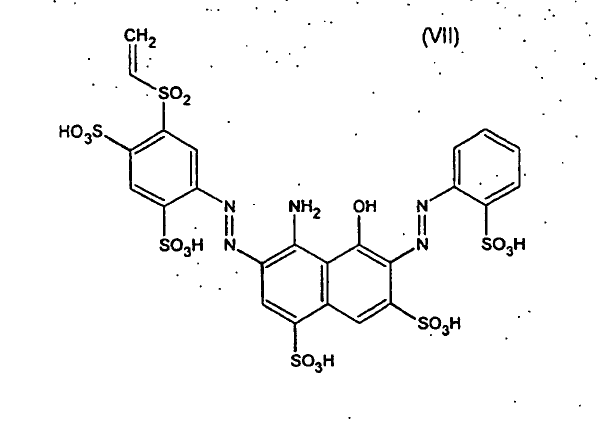 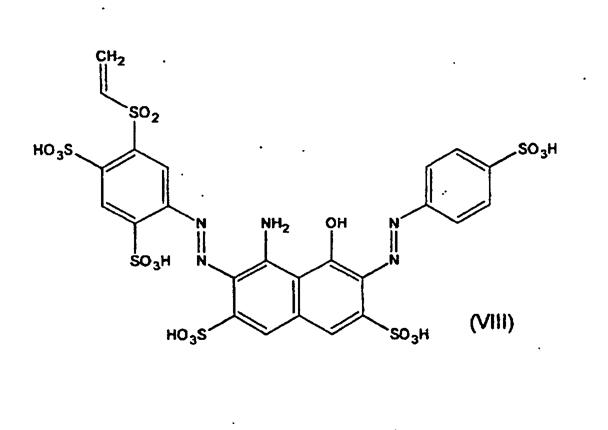 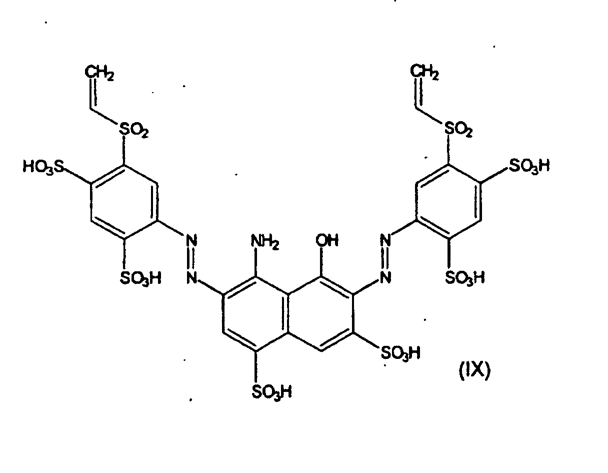 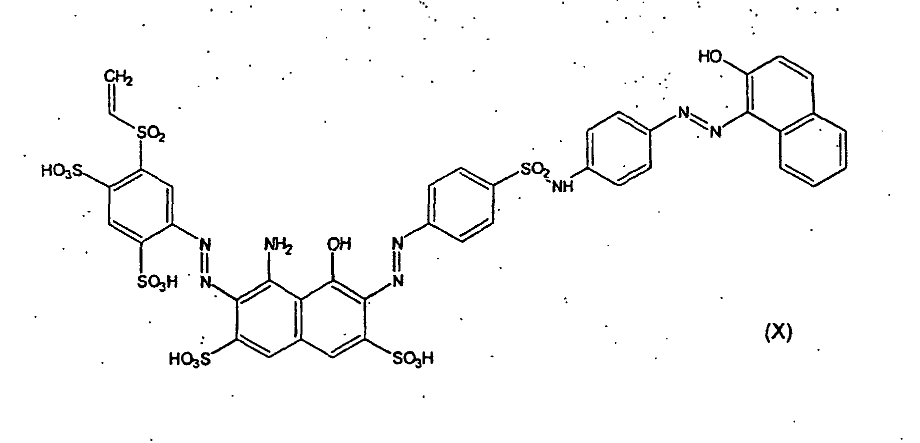 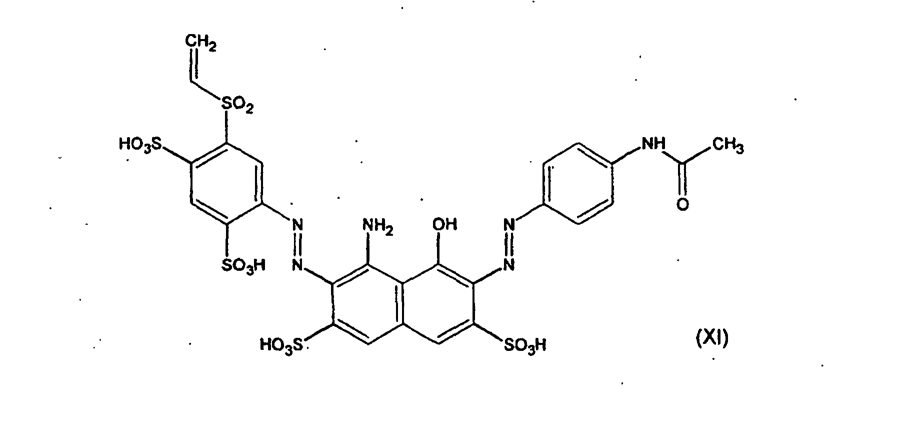 enthält.

3. Verfahren nach Anspruch 1 oder 2, **dadurch gekennzeichnet, daß** die in der Verfahrensstufe a) aufgebrachte Zusammensetzung mindestens einen Reaktivfarbstoff, der mindestens eine Vinylsulfongruppe aufweist, enthält.

4. Verfahren nach Anspruch 1 oder 2, **dadurch gekennzeichnet, daß** die in der Verfahrensstufe a) aufgebrachte Zusammensetzung mindestens einen Reaktivfarbstoff, der mindestens eine Sulfatoethylsulfongruppe aufweist, enthält.

5. Verfahren nach einem oder mehreren der Ansprüche 1 bis 4, **dadurch gekennzeichnet, daß** die in der Verfahrensstufe d) aufgebrachte alkalische Zusammensetzung einen pH-Wert von 8 bis 11 aufweist.

6. Verfahren nach einem oder mehreren der Ansprüche 1 bis 5, **dadurch gekennzeichnet, daß** die in der Verfahrensstufe d) aufgebrachte alkalische Zusammensetzung Ammoniak und/oder Mono-, Di- oder Triethanolamin enthält.

7. Verfahren nach einem oder mehreren der Ansprüche 1 bis 5, **dadurch gekennzeichnet, daß** die in der Verfahrensstufe d) aufgebrachte alkalische Zusammensetzung mindestens ein Alkalicarbonat und/oder Alkalihydrogencarbonat enthält.

## Claims

1. Process for the dyeing of human hair, **characterized by** the combination of the following steps:
a) Application onto human hair of a composition having a pH-value of 2 to 5, comprising at least one reactive dyestuff;
b) leaving to process between about 5 to 45 minutes;
c) optionally rinsing and drying;
d) application of an alkaline composition with a pH-value of 7.5 to 12, comprising at least one oxidation dyestuff precursor and a peroxide;
e) leaving to process for 1 to 20 minutes; and
f) rinsing or shampooing and drying the colored hair.

2. Process according to claim 1, wherein the composition applied in step a) comprises at least one reactive dyestuff of the following general formulae

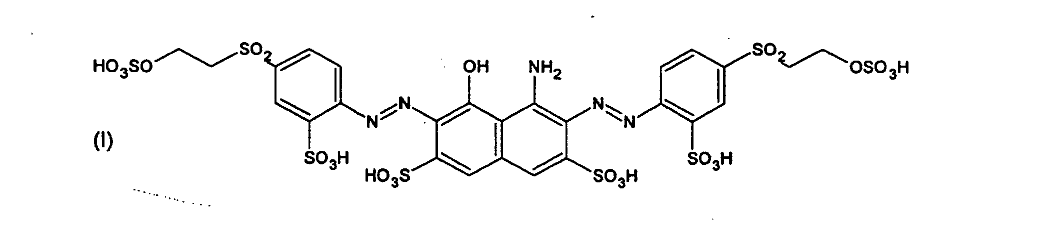

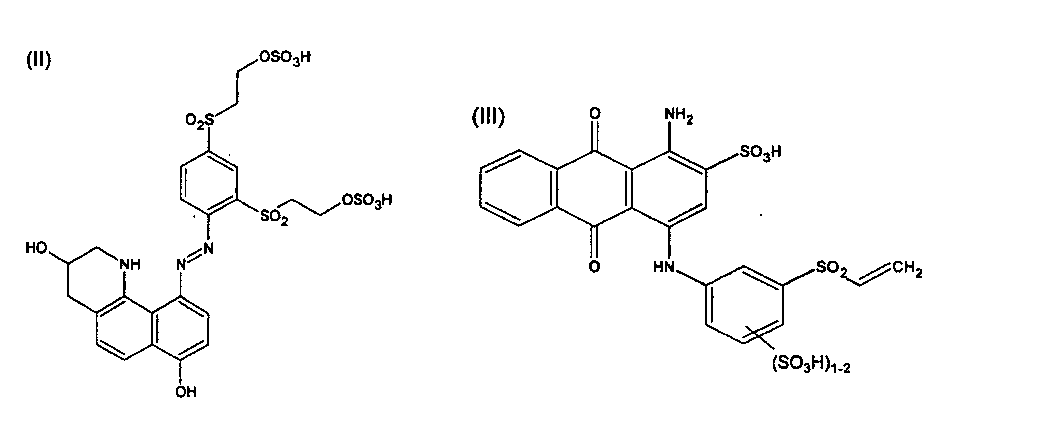

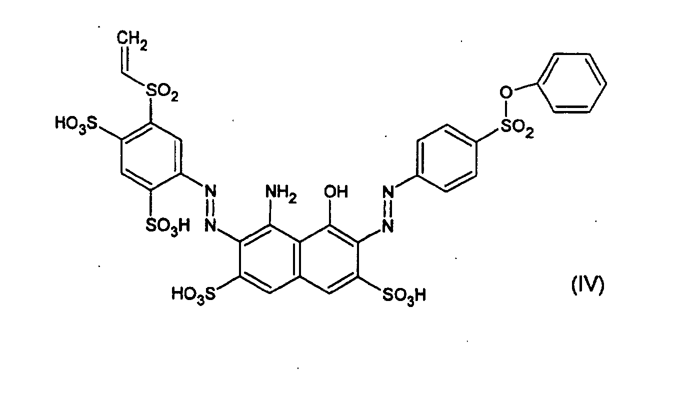

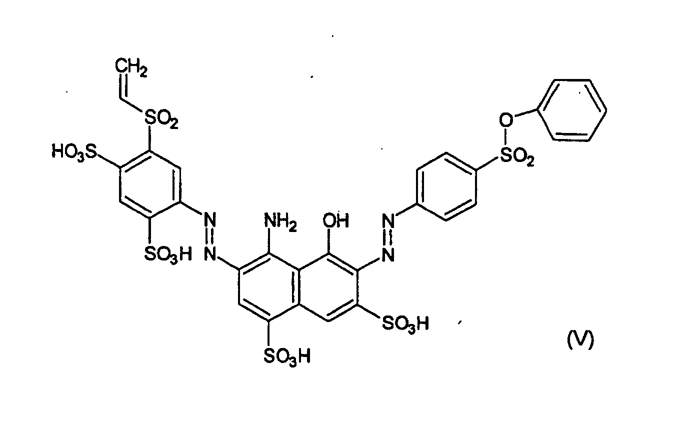

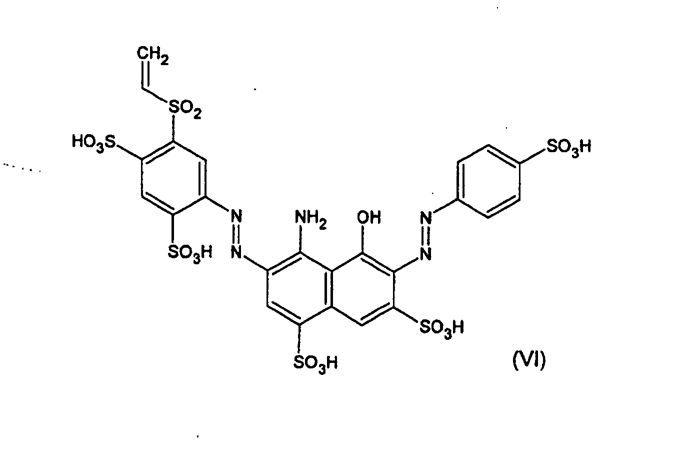

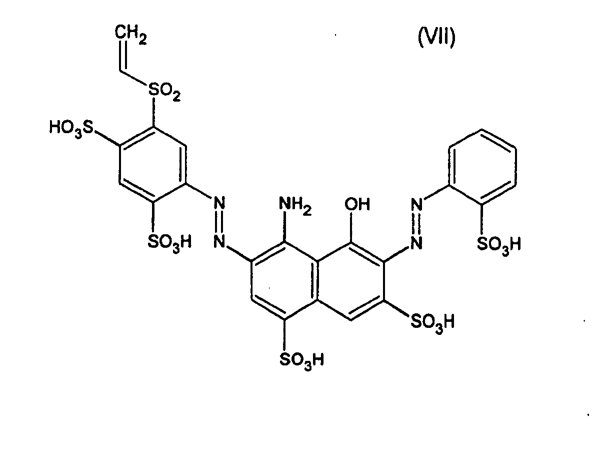

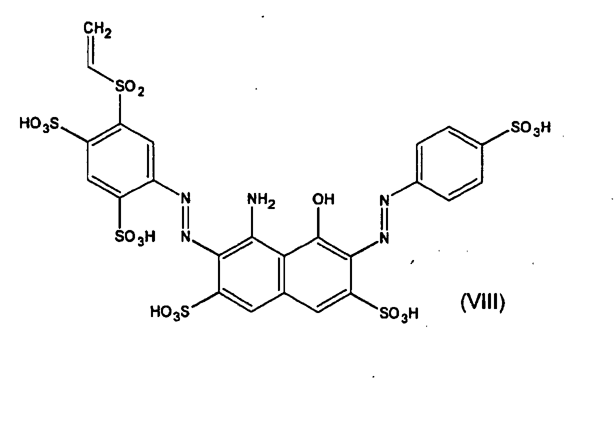

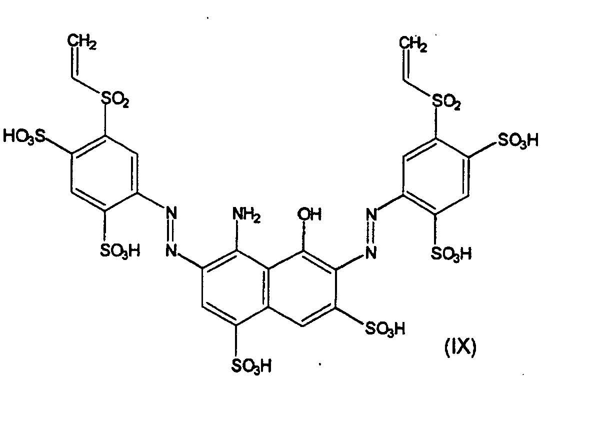

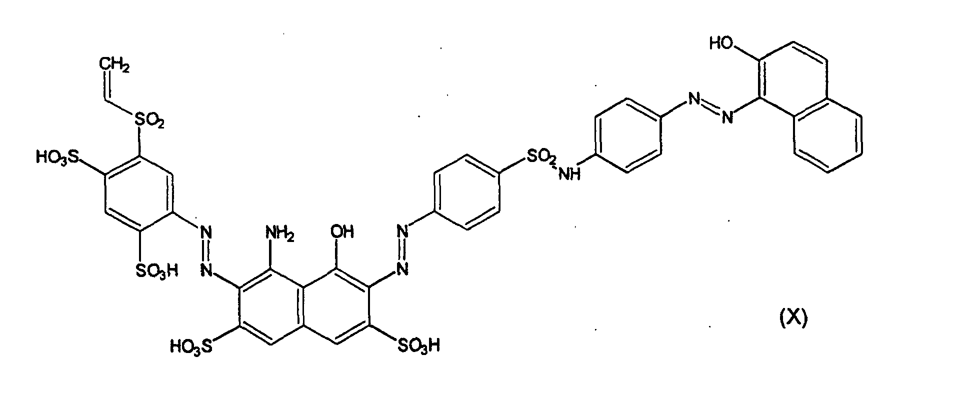

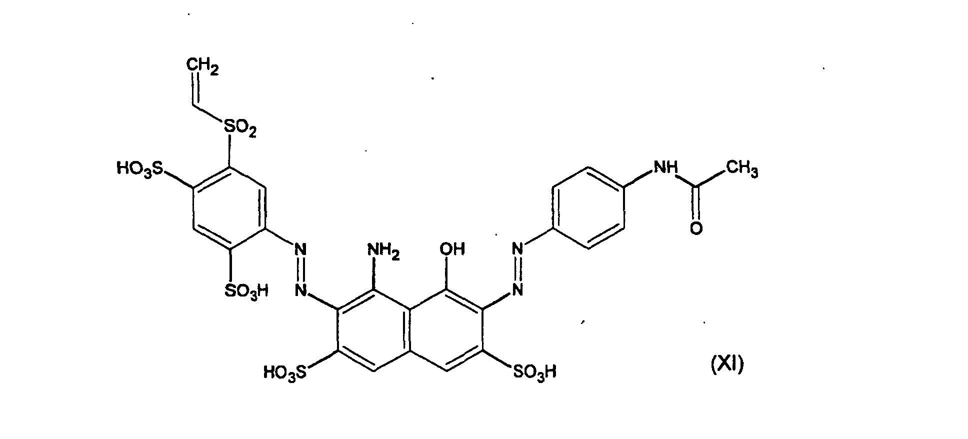

3. Process according to claim 1 or 2, wherein the composition applied in step a) comprises at least one reactive dyestuff having at least one vinyl sulfone group.

4. Process according to claim 1 or 2, wherein the composition applied in step a) comprises at least one reactive dyestuff bearing at least one sulfatoethyl sulfone group.

5. Process according to one or more of claims 1 to 4, wherein the alkaline composition applied in step d) has a pH-value from 8 to 11.

6. Process according to one or more of claims 1 to 5, wherein the alkaline composition applied in step d) comprises ammonia and/or mono-, di- or triethanolamine.

7. Process according to one or more of claims 1 to 5, wherein the alkaline composition applied in step d) comprises at least one alkali carbonate and/or alkali hydrogencarbonate.

## Revendications

1. Procédé de coloration de cheveux humains, **caractérisé par** la combinaison des étapes de procédé suivantes consistant à :
a) appliquer sur le cheveu humain une composition présentant un pH d'une valeur située entre 2 et 5, contenant au moins un colorant réactif ;
b) laisser agir environ de 5 à 45 minutes ;
c) le cas échéant, rincer et sécher ;
d) appliquer une composition alcaline contenant au moins précurseur de colorant d'oxydation et un peroxyde, et présentant un pH d'une valeur située entre 7,5 et 12 ;
e) laisser agir de 1 à 20 minutes ; et
f) rincer ou laver et sécher les cheveux colorés.

2. Procédé selon la revendication 1, **caractérisé en ce que** la composition appliquée au cours de l'étape a) du procédé contient au moins un colorant réactif des formules suivantes :

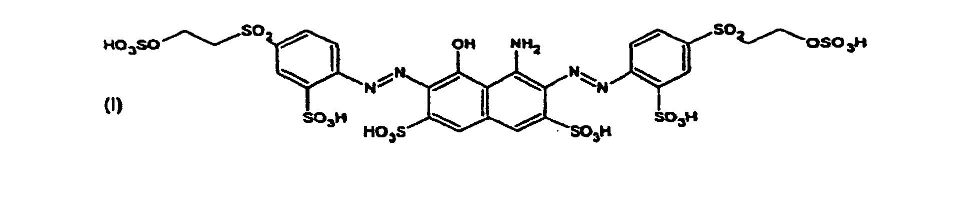

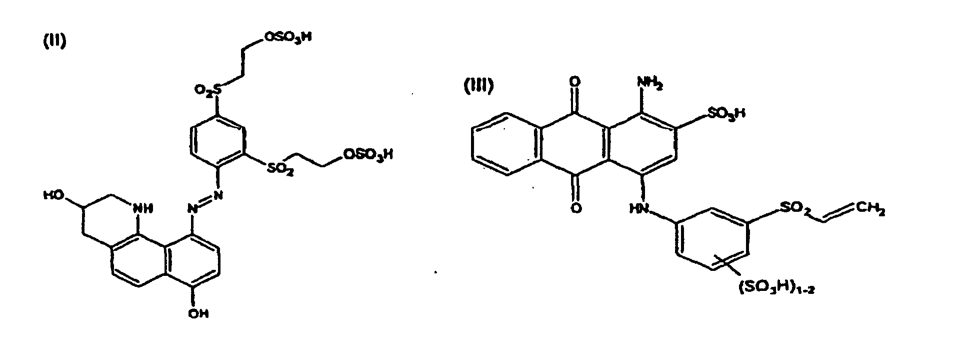

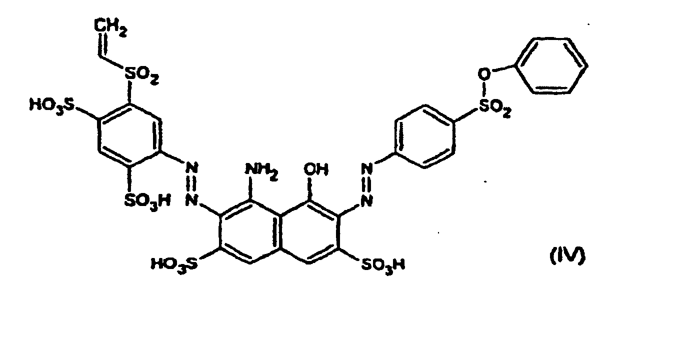

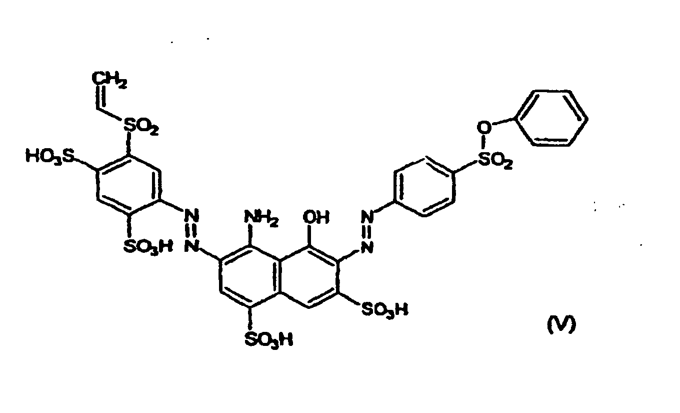

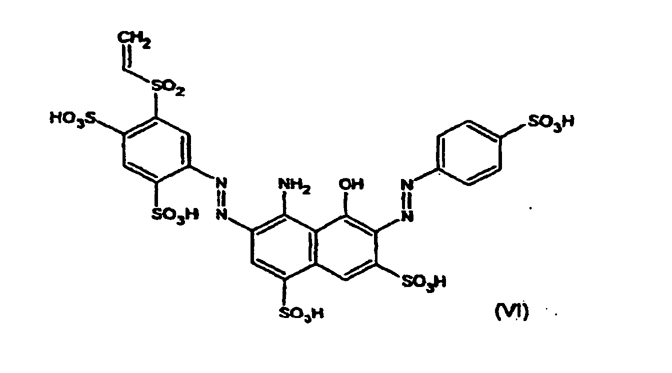

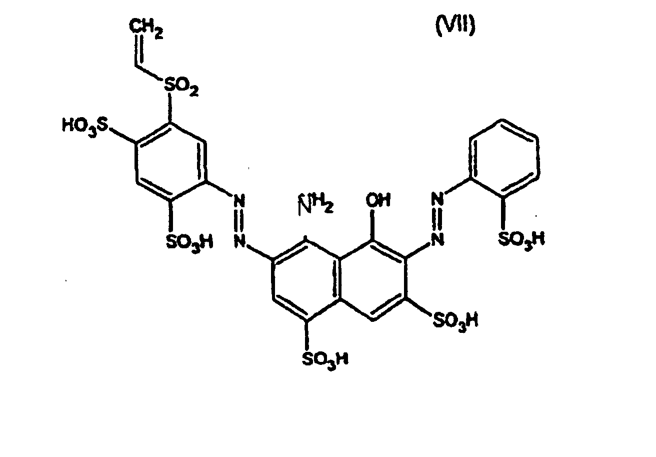

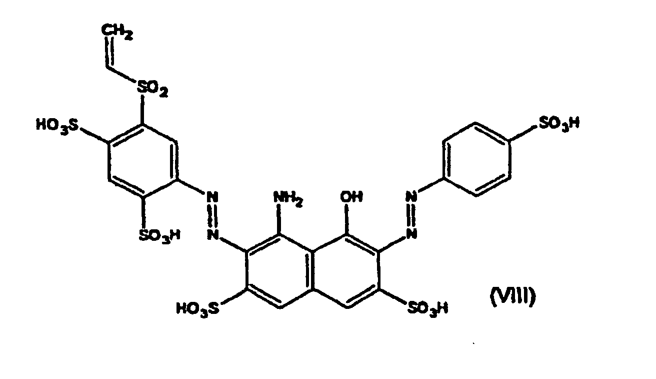

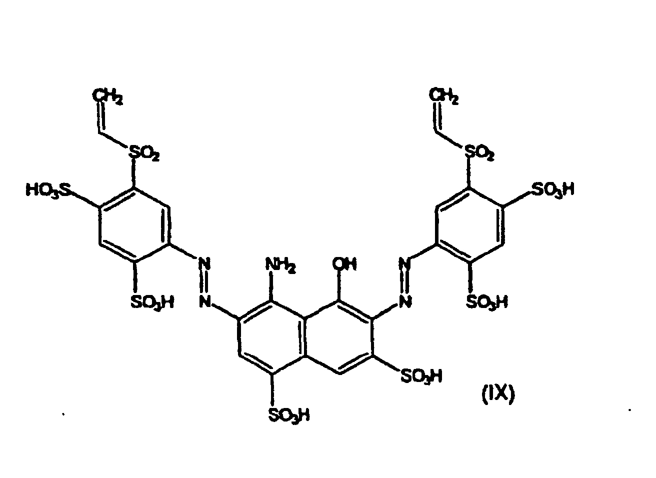

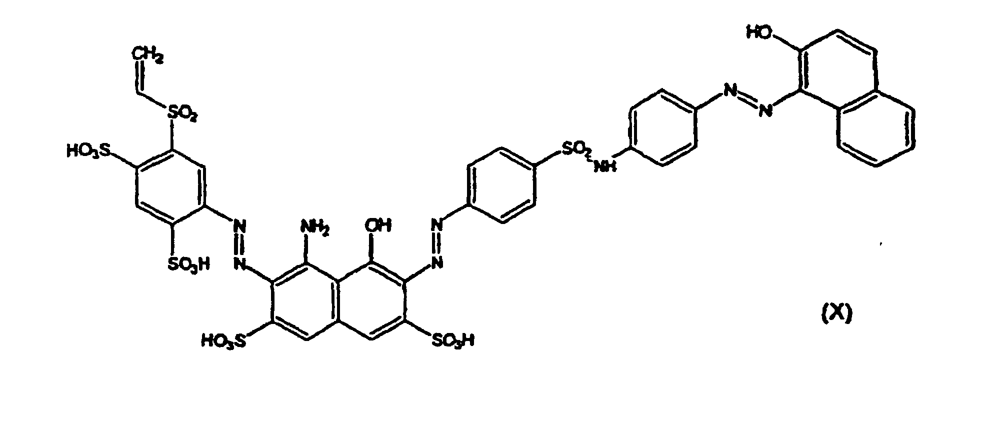

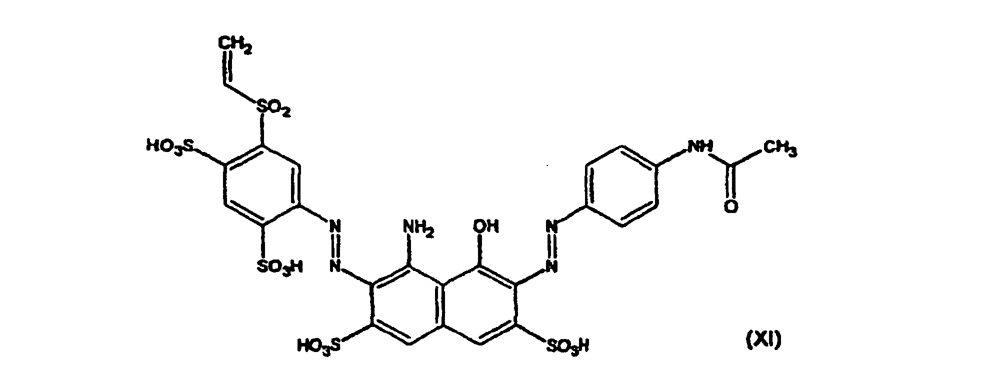

3. Procédé selon l'une quelconque des revendications 1 ou 2, **caractérisé en ce que** la composition qui est appliquée au cours de l'étape a) du procédé contient au moins un colorant réactif présentant au moins un groupe vinylsulfone.

4. Procédé selon l'une quelconque des revendications 1 ou 2, **caractérisé en ce que** la composition qui est appliquée au cours de l'étape a) du procédé contient au moins un colorant réactif présentant au moins un groupe de sulfatoéthylsulfone.

5. Procédé selon l'une quelconque ou plusieurs des revendications 1 à 4, **caractérisé en ce que** la composition alcaline appliquée lors de l'étape d) du procédé présente un pH d'une valeur située entre 8 et 11.

6. Procédé selon l'une quelconque ou plusieurs des revendications 1 à 5, **caractérisé en ce que** la composition alcaline appliquée lors de l'étape d) du procédé contient de l'ammoniaque et/ou une monoéthanolamine, une diéthanolamine ou une triéthanolamine.

7. Procédé selon l'une quelconque ou plusieurs des revendications 1 à 5, **caractérisé en ce que** la composition alcaline appliquée au cours de l'étape d) du procédé contient au moins un carbonate alcalin et/ un carbonate alcalin hydrogéné.
